(19) Europäisches Patentamt
European Patent Office
Office européen des brevets

(11) Veröffentlichungsnummer: **0 494 603 A2**

(12) # EUROPÄISCHE PATENTANMELDUNG

(21) Anmeldenummer: **92100050.1**

(22) Anmeldetag: **03.01.92**

(51) Int. Cl.[5]: **C07F 9/38**, C07D 207/08, C07C 309/14

(30) Priorität: **10.01.91 DE 4100533**

(43) Veröffentlichungstag der Anmeldung: **15.07.92 Patentblatt 92/29**

(84) Benannte Vertragsstaaten: **DE ES FR IT**

(71) Anmelder: **HOECHST AKTIENGESELLSCHAFT**
**Postfach 80 03 20**
**W-6230 Frankfurt am Main 80(DE)**

(72) Erfinder: **Tetzlaff, Heribert, Dr.**
**Geisenheimer Strasse 93**
**W-6000 Frankfurt am Main 71(DE)**
Erfinder: **Krull, Matthias, Dr.**
**Erlenweg 5**
**W-6232 Bad Soden am Taunus(DE)**
Erfinder: **Kremer, Gernot, Dr.**
**Frankfurter Strasse 200**
**W-6233 Kelkheim (Taunus)(DE)**
Erfinder: **Porz, Christoph. Dr.**
**Oppelnerstrasse 23**
**W-5309 Meckenheim(DE)**

(54) **Verfahren zur extrakfiven Abtrennung von Phospho- und Sulfobetainen aus sauren Reakfionslösungen.**

(57) Die Ampholyte werden mit einem flüssigen, mit der sauren, wäßrigen Reaktionslösung nicht mischbaren Kationenaustauscher aus der Reaktionslösung extrahiert, wobei der Kationenaustauscher in mindestens einem mit Wasser nicht mischbaren organischen Lösungsmittel gelöst ist und mit dem organischen Lösungsmittel eine organische Phase bildet. Anschließend wird die mit dem Ampholyten beladene organische Phase von der Reaktionslösung abgetrennt, und danach die Rückextraktion der Ampholyte aus der organischen Phase mit wäßrigen Lösungsmitteln durchgeführt. Das Verfahren arbeitet mit geringen Produktverlusten, der Restsalzgehalt ist sehr niedrig.

EP 0 494 603 A2

Die Erfindung betrifft ein Verfahren zur extraktiven Abtrennung von Ampholyten auf Basis von Phospho- und Sulfobetainen aus ihren sauren wäßrigen Reaktionslösungen. Ampholyte sind Verbindungen, die sowohl anionische als auch kationische Gruppen tragen.

Ampholyte von technischem Interesse sind zum Beispiel Phosphobetaine der Formel I

$R^1R^2YN^+-CHR^3-PO_3M^-$ (I),

in der $R^1$ und $R^2$ unabhängig voneinander Wasserstoff, $C_1$-$C_{20}$-Alkyl, $C_2$-$C_{20}$-Alkenyl, Alkylphenyl mit 1 bis 20 C-Atomen in der Alkylkette oder $C_1$-$C_{20}$-Acyl, vorzugsweise $C_1$-$C_{12}$-Alkyl, $C_2$-$C_{12}$-Alkenyl, Alkylphenyl mit 1 bis 12 C-Atomen in der Alkylkette oder $C_1$-$C_{12}$-Acyl, Y Wasserstoff oder $CHR^3$-$PO_3HM$, M Wasserstoff, $C_1$-$C_4$-Alkyl oder ein Kation, vorzugsweise Ammonium oder ein Alkalimetall-Kation, und $R^3$ Wasserstoff, Benzyl, $C_1$-$C_{10}$-Alkyl, vorzugsweise $C_1$-$C_5$-Alkyl oder $PO_3M$- bedeuten. Es ist auch möglich, daß die Phosphobetaine der Formel I ein Polyamin sind, in dem $R^2$ $[(CH_2)_nNZ]_xZ$, wobei n Zahlen von 1 bis 3 und x Zahlen von 2 bis 10 bedeuten, und Z die obenbeschriebene Bedeutung von $R^1$ oder Y haben kann.

Die Ampholyte der Formel I sind auf einfache Weise durch Umsetzung von Aminen der Formel $NHR^1R^2$ mit Aldehyden $R^3CHO$, wobei $R^1$, $R^2$ und $R^3$ die gleiche Bedeutung wie oben haben, und Phosphoriger Säure in einer Mannich-Reaktion zugänglich (K. Moedritzer, R.R. Irani, J. Org. Chem. 1966, 31, 1603). Dabei werden zur Verhinderung der Oxidation der Phosphorigen Säure und zur Erzielung hoher Ausbeuten bei kurzen Reaktionszeiten ein 2-3 molarer Überschuß an Mineralsäure wie z.B. Salz- oder Schwefelsäure zugegeben. Die gebildeten Phosphonsäuren können gegebenenfalls anschließend durch Neutralisation mit Basen in ihre Metallsalze überführt werden.

Bei der Synthese fallen die Phosphobetaine gewöhnlich in stark salzhaltigen Lösungen an, die zudem noch Edukte enthalten können.

Nach der nichtvorveröffentlichten deutschen Patentanmeldung P 40 01 420.7 gelingt die Isolierung der Aminomethylenphosphonsäuren der Formel I durch Ausfällen und gegebenenfalls mehrmaliges Umfällen aus geeigneten Lösungsmitteln. So erfolgt die Isolierung der Allylamino-bis(methylenphosphonsäure), in der $R^1$ $CH_2$-$CH=CH_2$, Y $CHR^3$-$PO_3HM$, $R^2$ und $R^3$ jeweils Wasserstoff bedeuten, dadurch, daß die Schwefelsäure aus der Reaktionsmischung mit Aceton extrahiert wird, wobei jedoch große Acetonmengen benötigt werden (12 x 3 l Aceton für 100 g Substanz) und erhebliche Produktmengen verloren gehen. Weiterhin ist bei diesem Verfahren eine Teilneutralisation der Reaktionslösung notwendig, die zu einem Sulfatrestgehalt von mehr als 6 Gew.-% im Produkt führt.

$\alpha$-Aminomethylenphosphonsäuren der Formel I finden Verwendung z.B. als Builder und Cobuilder in Waschmitteln (P 40 01 420.7), als Antikorrosionsreagenzien (DE-A-22 30 832 sowie DE-A-22 31 206) und in flammhemmenden Geweben (JP-A-48012475). Weiterhin werden sie als Komplexierungsreagenzien sowie zur Kristallmodifikation für die Steinverhinderung in salinen Wässern und beim Ölbohren eingesetzt (US-A-4 707 306). Für bestimmte Anwendungen müssen die Wirkstoffe dabei in Reinform, d.h. ohne Edukte wie z.B. Formaldehydreste, ohne Nebenprodukte und insbesondere salzfrei dargestellt werden.

Eine ähnliche Substanzklasse stellen die Sulfobetaine der Formeln II und III dar,

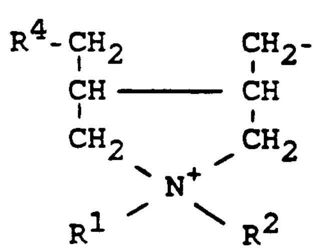 (II), $R^1(R^2)_2N^+-CH_2-CHR^5-CH_2-SO_3^-$ (III)

in der $R^1$ und $R^2$ unabhängig voneinander Wasserstoff, $C_1$-$C_{20}$-Alkyl, $C_2$-$C_{20}$-Alkenyl, Alkylphenyl mit 1 bis 20 C-Atomen in der Alkylkette oder $C_1$-$C_{20}$-Acyl, vorzugsweise $C_1$-$C_{12}$-Alkyl, $C_2$-$C_{12}$-Alkenyl, Alkylphenyl mit 1 bis 12 C-Atomen in der Alkylkette oder $C_1$-$C_{12}$-Acyl, bedeuten, in der $R^4$ Wasserstoff, $SO_2H$, $SO_2M$, wobei M Ammonium oder ein Alkalimetall-Kation ist, oder ein Sulfon der Formel $SO_2$-$R^6$ bedeutet, in der $R^5$ Wasserstoff, Hydroxyl, $SO_2H$, $SO_3H$ oder $SO_2R^6$ bedeutet, wobei $R^6$ für einen Rest der Gruppe $C_1$-$C_{30}$-Alkyl, vorzugsweise $C_1$-$C_{22}$-Alkyl, insbesondere Methyl oder Ethyl oder $C_{18}$-$C_{20}$-Alkyl, der gegebenenfalls mit Halogenen, vorzugsweise Fluor oder Chlor, Hydroxy- oder Aminogruppen substituiert und/oder wobei die Alkylkette an einer beliebigen Stelle durch die Gruppierung -CO-O-, -CO-NH-oder -NH- unterbrochen sein kann, steht.

Bei der Darstellung aus z.B. Diallyl-dimethyl-ammoniumchlorid und Sulfit gemäß EP-A-163 319 kristallisieren Sulfobetaine der Formel II ($R^1$ = $R^2$ = $-CH_3$, $R^4$ = H oder $SO_2H$) aus der salzsauren Lösung mit etwa 5 % Natriumchlorid. Auch hier ist für bestimmte Anwendungen eine salz- und nebenproduktfreie Isolierung erwünscht.

Aus DE-B-12 77 256 ist ein Verfahren zur Extraktion von Aminosäuren aus verdünnten wäßrigen Lösungen bei pH 4 bis 10 mit Natrium-Dinonylnaphthalinsulfonat bekannt, wobei der Extrakt mit wäßrigen Lösungen starker organischer Basen bzw. deren Salzen rückextrahiert wird. Über die Ionenaustauscher-Eigenschaften der Dinonylnaphthalinsulfonsäure geben E. Hoegfeldt et al. (Chem. Scr. 1981, 18, 13) einen Überblick.

Die Aufgabe der vorliegenden Erfindung besteht darin, ein gegenüber dem Stand der Technik weniger aufwendiges und möglichst verlustfreies Verfahren zur extraktiven Abtrennung von Ampholyten auf Basis von Phospho- und Sulfobetainen aus ihren sauren wäßrigen Reaktionslösungen zur Verfügung zu stellen, wobei der Restsalzgehalt im Produkt so gering wie möglich sein soll.

Es wurde nun überraschenderweise gefunden, daß Ampholyte auf Basis von Phospho- und Sulfobetainen, insbesondere mit den obengenannten Formeln (I), (II) und (III), mit einem flüssigen, mit der sauren wäßrigen Reaktionslösung nicht mischbaren Kationenaustauscher aus der Reaktionslösung extrahiert werden, wobei der Kationenaustauscher in mindestens einem mit Wasser nicht mischbaren organischen Lösungsmittel gelöst ist, und mit dem organischen Lösungsmittel eine organische Phase bildet, anschließend die mit den Ampholyten beladene organische Phase von der Reaktionslösung abgetrennt wird und danach die Rückextraktion der Ampholyte aus der organischen Phase mit wäßrigen Lösungsmitteln durchgeführt wird.

Die extrem hydrophilen Ampholyte werden dabei mit dem hydrophoben Kationenaustauscher in einer üblichen Extraktionsapparatur zweckmäßigerweise mehrstufig, insbesondere im Gegenstrom aus der sauren wäßrigen Reaktionslösung extrahiert. Anschließend wird die hydrophobe, mit dem Ampholyten beladene organische Phase von der wäßrigen Phase getrennt und in einer weiteren Extraktionsanlage zweckmäßigerweise ebenfalls mehrstufig und im Gegenstrom mit heißem Wasser rückextrahiert.

Für die Durchführung des erfindungsgemäßen Verfahrens ist es von besonderem Vorteil, daß die saure wäßrige Reaktionslösung einen pH-Wert von kleiner oder gleich 4, vorzugsweise kleiner oder gleich 2, aufweist. Als Kationenaustauscher werden insbesondere hydrophobe starke organische Säuren, vorzugsweise aromatische Carbon- oder Sulfonsäuren, die mit Alkylresten substituiert sind, verwendet. Besonders vorteilhaft ist es, wenn als Kationenaustauscher aromatische Carbon- und Sulfonsäuren, die 1 bis 3 Carboxy- oder Sulfogruppen, vorzugsweise eine Carboxy- oder Sulfogruppe, pro Molekül und 1 bis 4 Alkylketten enthalten, wobei die Anzahl der C-Atome der Carbon- oder Sulfonsäure insgesamt 10 bis 60, vorzugsweise 20 bis 40, insbesondere 25 bis 35 beträgt, oder Mischungen dieser Carbon- oder Sulfonsäuren verwendet werden. Der Kationenaustauscher ist in einem oder mehreren mit Wasser nicht mischbaren aliphatischen oder aromatischen Kohlenwasserstoffen oder einem oder mehreren mit Wasser nicht mischbaren aliphatischen oder aromatischen teilhalogenierten oder perhalogenierten Kohlenwasserstoffen, wobei Halogen Fluor und/oder Chlor ist, oder in Gemischen davon gelöst. Vorzugsweise werden Kerosin, Paraffine, Alkylaromaten oder Halogenkohlenwasserstoffe, die bei 20 °C zu weniger als 1 Gew.-% in Wasser löslich sind, verwendet. Die Konzentration des Kationenaustauschers in dem organischen Lösungsmittel kann 10 bis 90 Gew.-%, vorzugsweise 40 bis 60 Gew.-%, betragen.

Die extraktive Abtrennung der Ampholyte I bis III aus ihren sauren Lösungen als auch die Rückextraktion mit Wasser erfolgen am wirtschaftlichsten durch mehrstufige, kontinuierliche oder schubweise kontinuierliche Gegenstromextraktion. Als Apparate zur Durchführung der Extraktionen sind alle gängigen Extraktionsapparate geeignet, wie z.B. Kolonnen, Mischabsetzer oder Zentrifugalextraktoren.

In einer bevorzugten Ausführungsform des Verfahrens wird das Reaktionsgemisch (S) mehrstufig im Gegenstrom mit 50 Gew.-% Dinonylnaphthalinsulfonsäure in Kerosin (X) extrahiert. Die Extraktion findet zwischen 0 und 100 °C, bevorzugt zwischen 10 und 35 °C statt. Das Volumenverhältnis S:X liegt zwischen 1:10 und 10:1. Die am einen Ende der Extraktionsanlage ablaufende, produktfreie Mineralsäure kann nach Aufkonzentrierung wieder in die Reaktionsstufe zurückgeführt werden.

Der am anderen Ende der Extraktionsanlage ablaufende, mit dem Ampholyten beladene Extrakt wird einer weiteren Extraktionsanlage zugeführt, in der die erfindungsgemäßen Ampholyte in einer bevorzugten Variante des Verfahrens durch Behandlung mit heißem Wasser rückextrahiert werden. Die Rückextraktion mit Wasser (R) findet vorzugsweise mehrstufig im Gegenstrom statt. Die Temperaturen in der Rückextraktionskolonne liegen zwischen 40 und 100 °C, bevorzugt zwischen 60 und 100 °C und das Volumenverhältnis X:R liegt zwischen 1:10 und 10:1. Die Extraktphase wird anschließend wieder in die Extraktionsstufe zurückgeführt. Nach Eindampfen des wäßrigen Rückextrakts und gegebenenfalls Versetzen des Eindampfrückstandes mit geeigneten Lösungsmitteln wie z.B. Alkoholen, Ketonen oder Gemischen davon, können

die Verbindungen in kristalliner Form gewonnen werden.

Für die Rückextraktion werden also überraschenderweise keine Verdrängungsreagenzien wie die in DE-PS 12 77 256 verwendeten Amine bzw. Ammoniumsalze benötigt. Daher kann die Lösung des Kationenaustauschers direkt, d.h. ohne vorherige Aufarbeitung zur Extraktion wiederverwendet werden.

Das Verfahren wird anhand der folgenden Beispiele näher erläutert.

Beispiel 1

Eine Reaktionslösung mit 32 Gew.-% Allylaminobismethylenphosphonsäure in konzentrierter Schwefelsäure wird im Labormischabsetzer mit der 6-fachen Menge an 50 Gew.-% Dinonylnaphthalinsulfonsäure in Kerosin 10-stufig im Gegenstrom extrahiert. Dabei gehen 99 % der Phosphonsäure in die organische Phase über. Anschließend wird die organische Extraktphase ebenfalls im Labormischabsetzer bei 90 °C mit 1/6 der Menge an Wasser rückextrahiert, wobei die Phosphonsäure praktisch quantitativ in die wäßrige Phase übergeht. Die verbleibende organische Phase kann wieder zur Extraktion eingesetzt werden. Nach Abdampfen des Wassers bei 40 °C/l mbar wird die verbleibende Masse mit der 3-fachen Menge Ethanol umkristallisiert und nach Abfiltrieren im Vakuum getrocknet. Die Ausbeute an Phosphonsäure bezogen auf die ursprüngliche Menge in der Reaktionslösung beträgt 85 %, der Sulfatgehalt 1,0 Gew.-%. Bei Wiederverwendung des zur Umkristallisation eingesetzten Ethanols kann die Ausbeute weiter erhöht werden.

Beispiel 2

Eine schwefelsaure Reaktionslösung mit 35 Gew.-% Diallylaminomethylenphosphonsäure (Phosphobetain) wird in einer Schwingbodenkolonne (Typ Karr, 3 m hoch, ⌀ = 50 mm) bei Raumtemperatur mit der gleichen Menge Dinonylnaphthalinsulfonsäure (50 Gew.-% in Kerosin) kontinuierlich im Gegenstrom extrahiert. 90 % des Phosphobetains gehen dabei in die organische Extraktphase über. Der organische Extrakt wird anschließend in eine zweite, baugleiche Schwingbodenkolonne gepumpt, wo er bei 80 °C kontinuierlich mit der gleichen Menge Wasser rückextrahiert wird. Das Phosphobetain, das dabei in die wäßrige Phase übergeht, wird durch Eindampfen im Vakuum (40 °C, 1 mbar) als gelbliches Öl gewonnen.

Die Gesamtausbeute an Diallylaminomethylenphosphonsäure beträgt 85 % der Einsatzmenge.

Beispiel 3

Eine salzsaure Reaktionslösung (pH 2) mit 30 Gew.-% des Sulfobetains II ($R^1 = R^2 = CH_3$, $R^4 = SO_2$-$CH_2$-COOH) wird im Labormischabsetzer bei Raumtemperatur mit der gleichen Menge Dinonylnaphthalinsulfonsäure (50 Gew.-% in Kerosin) kontinuierlich einer 8-stufigen Gegenstromextraktion unterzogen. Dabei gehen 95 % des Sulfobetains in die organische Phase über. Der organische Extrakt wird anschließend kontinuierlich in einen zweiten Labormischabsetzer gefördert, wo das Sulfobetain II bei 60 °C mit der gleichen Menge Wasser rückextrahiert wird (kontinuerlich, 8 Stufen). Das Sulfobetain geht dabei praktisch quantitativ in die wäßrige Phase über, die organische Phase wird in die Extraktionsstufe zurückgeführt. Der Rückextrakt wird bis zum Verbleib einer zähflüssigen Masse bei 40 °C, 1 mbar eingeengt und das Sulfobetain daraus durch Zugabe zu kaltem Ethanol gefällt. Nach abfiltrieren und trocknen (40 °C, Vakuumtrockenschrank) erhält man das Sulfobetain in 80 %-iger Ausbeute mit einem Rest-Chloridgehalt von 0,74 %.

Diese Beispiele zeigen, daß bei dem erfindungsgemäßen Verfahren die Verlustraten an Produkt relativ gering sind und der Sulfatrestgehalt den aus dem Stand der Technik bekannten deutlich unterschreitet.

## Patentansprüche

1. Verfahren zur extraktiven Abtrennung von Ampholyten auf Basis von Phospho- und Sulfobetainen aus ihren sauren wäßrigen Reaktionslösungen, dadurch gekennzeichnet, daß die Ampholyte mit einem flüssigen, mit der sauren, wäßrigen Reaktionslösung nicht mischbaren Kationenaustauscher aus der Reaktionslösung extrahiert werden, wobei der Kationenaustauscher in mindestens einem mit Wasser nicht mischbaren organischen Lösungsmittel gelöst ist und mit dem organischen Lösungsmittel eine organische Phase bildet, anschließend die mit dem Ampholyten beladene organische Phase von der Reaktionslösung abgetrennt wird und danach die Rückextraktion der Ampholyte aus der organischen Phase mit wäßrigen Lösungsmitteln durchgeführt wird.

**2.** Verfahren nach Anspruch 1, dadurch gekennzeichnet, daß die saure, wäßrige Reaktionslösung einen pH-Wert von kleiner oder gleich 4, vorzugsweise kleiner oder gleich 2, aufweist.

**3.** Verfahren nach einem der Ansprüche 1 oder 2, dadurch gekennzeichnet, daß Kationenaustauscher auf Basis von hydrophoben starken organischen Säuren, vorzugsweise aromatischen Carbon- oder Sulfonsäuren, die mit Alkylresten substituiert sind, verwendet werden.

**4.** Verfahren nach einem der Ansprüche 1 bis 3, dadurch gekennzeichnet, daß als Kationenaustauscher aromatische Carbon- und Sulfonsäuren, die 1 bis 3 Carboxy- oder Sulfogruppen, vorzugsweise eine Carboxy- oder Sulfogruppe, pro Molekül und 1 bis 4 Alkylketten enthalten, wobei die Anzahl der C-Atome der Carbon- oder Sulfonsäure insgesamt 10 bis 60, vorzugsweise 20 bis 40, insbesondere 25 bis 35 beträgt, oder Mischungen dieser Carbon- oder Sulfonsäuren verwendet werden.

**5.** Verfahren nach einem der Ansprüche 1 bis 4, dadurch gekennzeichnet, daß der Kationenaustauscher in einem organischen Lösungsmittel auf Basis von einem oder mehreren mit Wasser nicht mischbaren aliphatischen oder aromatischen Kohlenwasserstoffen oder einem oder mehreren mit Wasser nicht mischbaren aliphatischen oder aromatischen teilhalogenierten oder perhalogenierten Kohlenwasserstoffen, wobei Halogen Fluor und/oder Chlor ist, oder in Gemischen davon gelöst ist.

**6.** Verfahren nach einem der Ansprüche 1 bis 5, dadurch gekennzeichnet, daß die Extraktion bei Temperaturen zwischen 0 und 100 °C, vorzugsweise zwischen 10 und 35 °C, durchgeführt wird.

**7.** Verfahren nach einem der Ansprüche 1 bis 6, dadurch gekennzeichnet, daß die Extraktion mehrstufig, bevorzugt im Gegenstrom durchgeführt wird.

**8.** Verfahren nach einem der Ansprüche 1 bis 7, dadurch gekennzeichnet, daß der Ampholyt mit wäßrigen Lösungsmitteln vom Kationenaustauscher bei 40 bis 100 °C, vorzugsweise 80 bis 100 °C, rückextrahiert wird und danach gegebenenfalls der erhaltene Ampholyt kristallisiert wird.

**9.** Verfahren nach einem der Ansprüche 1 bis 8, dadurch gekennzeichnet, daß die Konzentration des Kationenaustauschers in dem organischen Lösungsmittel 10 bis 90 Gew.-%, bevorzugt 40 bis 60 Gew.-%, beträgt.

**10.** Verfahren nach einem der Ansprüche 1 bis 9, dadurch gekennzeichnet, daß die Phosphobetaine die Formel I

$$R^1R^2YN^+\text{-}CHR^3\text{-}PO_3M^- \quad (I)$$

aufweisen, in der $R^1$ und $R^2$ unabhängig voneinander Wasserstoff, $C_1$-$C_{20}$-Alkyl, $C_2$-$C_{20}$-Alkenyl, Alkylphenyl mit 1 bis 20 C-Atomen in der Alkylkette oder $C_1$-$C_{20}$-Acyl, vorzugsweise $C_1$-$C_{12}$-Alkyl, $C_2$-$C_{12}$-Alkenyl, Alkylphenyl mit 1 bis 12 C-Atomen in der Alkylkette oder $C_1$-$C_{12}$-Acyl, oder in dem $R^2$ eine Gruppe der Formel $[(CH_2)_nNZ]_xZ$, wobei n Zahlen von 1 bis 3 und x Zahlen von 2 bis 10 sind und Z die Bedeutung von $R^1$ oder Y haben kann, Y Wasserstoff oder $CHR^3$-$PO_3HM$, M Wasserstoff, $C_1$-$C_4$-Alkyl oder ein Kation, vorzugsweise Ammonium oder ein Alkalimetall-Kation, und $R^3$ Wasserstoff, Benzyl oder $C_1$-$C_{10}$-Alkyl, vorzugsweise $C_1$-$C_5$-Alkyl, bedeuten.

**11.** Verfahren nach einem der Ansprüche 1 bis 9, dadurch gekennzeichnet, daß die Sulfobetaine die Formel II oder III

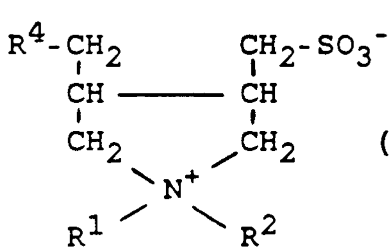 (II), $R^1(R^2)_2N^+\text{-}CH_2\text{-}CHR^5\text{-}CH_2\text{-}SO_3^-$ (III)

aufweisen, in der $R^1$ und $R^2$ unabhängig voneinander Wasserstoff, $C_1$-$C_{20}$-Alkyl, $C_2$-$C_{20}$-Alkenyl, Alkylphenyl mit 1 bis 20 C-Atomen in der Alkylkette oder $C_1$-$C_{20}$-Acyl, vorzugsweise $C_1$-$C_{12}$-Alkyl, $C_2$-$C_{12}$-Alkenyl, Alkylphenyl mit 1 bis 12 C-Atomen in der Alkylkette oder $C_1$-$C_{12}$-Acyl, bedeuten, in der $R^4$ Wasserstoff, $SO_2H$, $SO_2M$, wobei M Ammonium oder ein Alkalimetall-Kation ist, oder ein Rest der Formel $SO_2$-$R^6$ bedeutet, in der $R^5$ Wasserstoff, Hydroxyl, $SO_2H$, $SO_3H$ oder $SO_2$-$R^6$ bedeutet, wobei $R^6$ für einen Rest der Gruppe $C_1$-$C_{30}$-Alkyl, vorzugsweise $C_1$-$C_{22}$-Alkyl, insbesondere Methyl oder Ethyl oder $C_{18}$-$C_{20}$-Alkyl, der gegebenenfalls mit Halogenen, vorzugsweise Fluor oder Chlor, Hydroxy- oder Aminogruppen substituiert und/oder wobei die Alkylkette an einer beliebigen Stelle durch die Gruppierung -CO-O-, -CO-NH-oder -NH- unterbrochen sein kann, steht.

**12.** Ampholyte auf Basis von Phospho- und Sulfobetainen, dadurch gekennzeichnet, daß sie durch das Verfahren nach einem der Ansprüche 1 bis 9 erhalten werden.